# EUROPEAN PATENT SPECIFICATION

(11) **EP 0 492 484 B1**
(45) Date of publication and mention of the grant of the patent: **09.11.1994**
(21) Application number: 91121881.6
(22) Date of filing: 23.12.1991
(51) Int. Cl.: C07D 295/194, A61K 31/535

(54) **Substituted N-[phenyl-(thioxomethyl)]-morpholine derivatives, a process for preparing them as well as compositions containing these compounds and the use of them**
Substituierte N-[Phenyl-(thioxomethyl)]-morpholinderivate, Verfahren zu ihrer Herstellung, diese enthaltende Zusammensetzung und ihre Verwendung
N-[phenyl-(thioxomethyl)]-morpholine derivatives substitués procédé pour leur préparation aussi bien que compositions les contenant et leur utilisation

(30) Priority: 22.12.1990 HU 844790
(43) Date of publication of application: 01.07.1992
(73) Proprietor: EGIS GYOGYSZERGYAR, Budapest X (HU)
(72) Inventor: Budai, Zoltan, Dr., H-Budapest II (HU); Mezei, Tibor, Dr., H-1221 Budapest (HU); Reiter, Klára, Dr., H-1022 Budapest (HU); Fekete, Lajos, Dr., H-1165 Budapest (HU); Magyar, Károly, Dr., H-1012 Budapest (HU); Nagy, Attila, Dr., H-1031 Budapest (HU); Puskás, Lászl , Dr., H-Budapest 113 (HU)
(74) Representative: Beszédes, Stephan G., Dr.

(56) References cited:
- PATENT ABSTRACTS OF JAPAN vol. 5, no. 62 (C-52)(734) 25 April 1981
- EUR.J.MED. CHEM.-CHIMICA THERAPEUTICA vol. 14, no. 1, January 1979, pages 27 - 31; C.FARINA ET AL.: 'New antisecretory and antiulcer alkoxythiobenzamides .II'
- MARTI NEGWER 'Organic-chemical drugs and their synonyms' 1987 , AKADEMIE-VERLAG , BERLIN

## Description

This invention is concerned with new substituted N-[phenyl-(thioxomethyl)]-morpholine derivatives, a process for preparing them as well as compositions containing these compounds and the use of them.

It is known that 4-[3'-(methoxy)-4'-(hydroxy)-thiobenzoyl]-morpholine possesses choleretic activity (Martin Negwer: "Organic-chemical drugs and their synonyms", Akademie-Verlag; Berlin, 1987).

It is also known that 4-[3',4',5'-tri-(methoxy)-thiobenzoyl]-morpholine and 4-{[3',5'-di-(methoxy)-4'-[hydroxy)-phenyl]-thioxomethyl}-morpholine exert ulcus secretion inhibiting properties (Farina, C., Pinza, M., Gomba, A. and Pifferi, G.: Eur. J. Med. Chem. Chim. Ther. 14 [1979], pages 27 to 31).

Furthermore from US-PS 4,489,074 4-{[3',4'-di-(methoxy)-phenyl]-thioxomethyl}-morpholine, 4-{[3',5'-di-(methoxy)-phenyl]-thioxomethyl}-morpholine and 4-{[3',4',5'-tri-(methoxy)-phenyl]-thioxomethyl}-morpholine have been known as intermediary products.

Moreover from PATENT ABSTRACTS OF JAPAN, vol. 5, No. 62 (C-52) (734) N-(3-methoxy-4-hydroxythiobenzoyl)-morpholine has been known as having gastric juice secretion suppressing effect and remedying effect to the acute and the chronic ulcer of the stomach and the duodenal.

From DE-OS 21 02 246 4-{[3',5'-di-(methoxy)-4'-(ethoxy)-phenyl]-thioxomethyl}-morpholine and 4-{3',4',5'-tri-(methoxy)-phenyl]-thioxomethyl}-morpholine have been known as having activity on the central nervous system.

The problem underlying to the invention is to create new substituted N-[phenyl-(thioxomethyl)]-morpholine derivatives which have superior properties of increasing the weight gain and improving feed utilization of domestic animals, a process for preparing them as well as compositions containing these compounds and the use of them.

Surprisingly this has been solved by the invention.

A subject matter of the invention are substituted N-[phenyl-(thioxomethyl)]-morpholine derivatives of the general formula 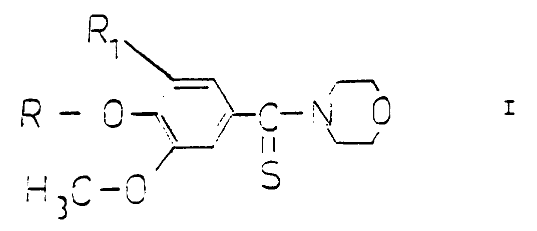
wherein
- R: represents hydrogen, C₁₋₁₈ alkyl, C₂₋₆ alkenyl or C₂₋₆ alkynyl and
- R₁: stands for hydrogen, halogen or methoxy,
with the provisos that
a) at least one of R and R₁ is other than hydrogen,
b) if R represents ethyl
   R₁ is other than hydrogen or methoxy and
c) if R stands for methyl
   R₁ is other than hydrogen or methoxy and
d) if R stands for hydrogen
   R₁ is other than methoxy,
and acid addition salts thereof.

Preferably the C₁₋₁₈ alkyl group which may be repre-sented by R is such having from 4 to 12, particularly from 6 to 12, most particularly 6, carbon atoms. From these straight chained groups are preferred.

Preferably the C₂₋₆ alkenyl or C₂₋₆ alkynyl group which may be represented by R is such having from 3 to 5, particularly 3 or 4, most particularly 3, carbon atoms. Also in this case straight chained groups are preferred. Most preferred the alkenyl group is a propenyl group and the alkynyl group a propynyl group.

The term "C₁₋₁₈ alkyl" used throughout the specification relates to straight or branched chained saturated aliphatic groups having the given number of carbon atoms, e.g. methyl, ethyl, n-propyl, isopropyl, n-butyl, n-hexyl, n-dodecyl and n-hexadecyl. "C₂₋₆ alkenyl" is defined as straight or branched chained alkenyl groups having the given number of carbon atoms, e.g. vinyl, allyl and propenyl. The term "C₂₋₆ alkynyl" relates to straight or branched chained alkynyl groups, e.g. propynyl. The term "halogen" encompasses all the four halogen atoms, such as fluorine, chlorine, bromine and iodine.

A particularly advantageous representative of the compounds according to the invention is 4-{[4'-(n-hexyloxy)-3'-(methoxy)-phenyl]-thioxomethyl}-morpholine.

According to a further aspect of the present inven-tion there is provided a process for preparing N-[phenyl-(thioxomethyl)]-morpholine derivatives according to the invention, characterized by
a) reacting a substituted benzaldehyde of the general formula 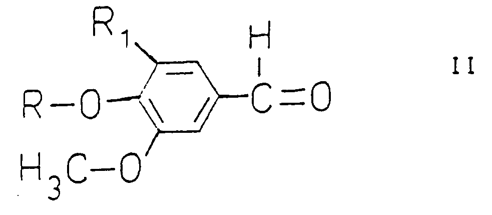 wherein
   R and R₁ are as stated above, with morpholine and sulphur, or
b) reacting a substituted thiobenzoic acid of the general formula 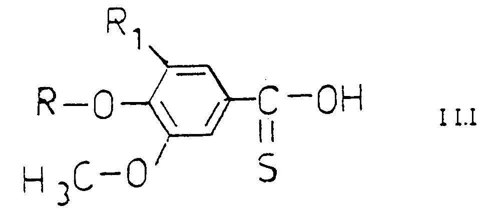 wherein
   R and R₁ are as stated above, or a reactive derivative thereof with morpholine or
c) reacting a morpholide of the general formula 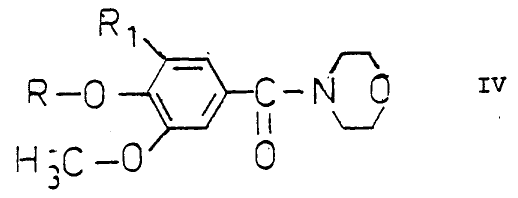 wherein
   R and R₁ are as stated above,
with a thionating agent
and, if desired, in a compound of the general formula I in which R represents hydrogen, obtained according to any of process variants a) to c) alkylating, alkenylating or alkynylating the respective hydroxy group, particularly by reacting a compound of the general formula I, wherein R stands for hydrogen, obtained according to any of process variants a) to c) with a halogenide of the general formula

R₂-Hlg V

wherein
- Hlg: represents halogen and
- R₂: stands for C₁₋₁₈ alkyl, C₂₋₆ alkenyl or C₂₋₆ alkynyl,
and/or, if desired, in a manner known per se, converting the obtained compound of the general formula I into an acid addition salt or if desired, converting the acid addition salt of the compound of formula I into the free base of formula I or into another acid addition salt.

In variant a) of the process of the invention for the reaction of the aldehyde of the general formula II with morpholine and sulphur advantageously the molar ratio of the aldehyde, morpholine and sulphur is chosen to be 1 : 2 : 1 , but for a good reaction also one of them may be applied in a slight excess.

Suitably the reaction is performed at elevated temperatures. Preferably a reaction temperature of 40°C to 160°C, particularly about 120°C, is applied. Furthermore it is preferred to carry out the reaction without using any solvent, in melt.

The time of the reaction depends on the temperature and may vary between wide ranges. If the reaction is carried out at a relatively low temperature, e.g. at 60°C, the time of reaction may be as long as 50 to 60 hours. At a relatively high temperature, e.g. 140°C, the reaction may be accomplished within 0.5 to 0.8 hour. At 120°C the optimum reaction time is 1 hour.

The substituted N-[phenyl-(thioxomethyl)]-morpholine derivatives of the general formula I can be isolated from the reaction mixture by known methods after adding a solvent, e.g. by crystallization after cooling or by evaporation.

In variant b) of the process of the invention the reaction of the substituted thiobenzoic acid of the general formula III with morpholine is preferably carried out with the former after having been activated to a reactive derivative.

The activation of the substituted thiobenzoic acid of the general formula III can be carried out in different ways. It is preferable to convert it into the corresponding halide with a halogenating agent, such as phosphorus pentachloride, phosphorus trichloride oxide or thionyl chloride. The halide thus obtained then may be reacted with the morpholine in the presence of an acid-binding agent. The reaction is preferably carried out in a solvent inert toward the reactants. As solvents aromatic hydrocarbons, e.g. toluene, benzene and/or [a] xylene(s), and/or ethers, such as tetrahydrofurane and/or diisopropyl ether, can be used. The morpholine used as reagent may serve as acid-binding agent, but organic and/or inorganic bases can also be applied for the same purpose. As organic base e.g. piperazine, triethylamine and/or ammonia, and as inorganic base e.g. 1 or more alkali hydroxide(s), carbonate(s) and/or hydrogen carbonate(s) may be used. As reactive acid derivatives anhydrides, mixed anhydrides, e.g. chloroformates, or esters, e.g. alkyl esters, may also be used. Thus according to a preferred embodiment of the process according to the invention as a reactive derivative of the substituted thiobenzoic acid of the general formula III an acid halide, acid anhydride, mixed anhydride or ester is used the first one being particularly preferred.

In variant c) of the process according to the invention for the reaction of the morpholide derivative of the general formula IV with the thionating agent as the latter preferably diphosphorus pentasulfide is used. Furthermore it is preferred to carry out this reaction in a solvent inert toward the reactants, particularly in pyridine, at an elevated temperature, particularly at temperatures from 40 to 160°C, most particularly at the boiling point of the solvent applied.

Preferably the optional alkylation, alkenylation or alkynylation, respectively, of a compound of the general formula I, wherein R stands for hydrogen, obtained according to any of process variants a) to c) with the halogenide of the formula V is carried out with the corresponding chloride or bromide as halogenide by methods known per se. Moreover it is preferred to work in the presence of a basic condensing agent. For this purpose suitably 1 or more alkali carbonate(s), e.g. sodium and/or potassium carbonate, alkali hydrogen carbonate(s) and/or alkali alcoholate(s) can be used. Particularly preferred is the use of potassium carbonate. The reaction is preferably performed under heating. It is particularly preferable to keep the reaction mixture boiling while using a reflux condenser.

The substituted benzaldehydes of the general formula II used as starting compounds are known from the literature (J. Prakt. Chem. 318 [1976] (5), pages 785 to 794). The substituted thiobenzoic acid derivatives of the general formula III used as starting materials can be prepared as described in US patent specification No. 1,855,454. The morpholides of the general formula IV used as starting materials can be prepared according to the method of Example 3.

As already mentioned, the compounds of the invention possess useful weight-gain increasing properties on domestic animals, particularly on pig, accompanied by a valuable fodder-utilization improving effect.

Hence by the invention also there are provided for compositions for use in animal husbandry, characterized in that they contain as [an] active ingredient(s) 1 or more N-[phenyl-(thioxomethyl)]-morpholine derivative(s) of the general formula 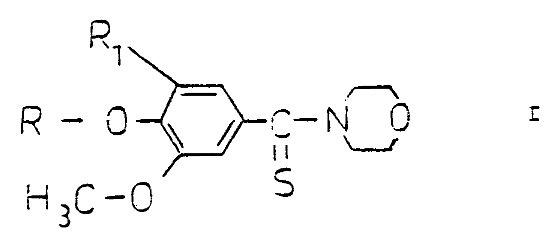
wherein
- R: represents hydrogen, C₁₋₁₈ alkyl, C₂₋₆ alkenyl or C₂₋₆ alkynyl and
- R₁: stands for hydrogen, halogen or methoxy,
with the provisos that
a) at least one of R and R₁ is other than hydrogen,
b) if R represents ethyl
   R₁ is other than hydrogen and
c) if R stands for methyl
   R₁ is other than hydrogen or methoxy,
and acid addition salts thereof, suitably in admixture with 1 or more inert solid and/or liquid carrier(s) and/or diluent(s) and optionally also 1 or more additive(s) generally used in the production of fodder additives and fodders.

The weight-gain increasing and fodder-utilization improving effects of the compounds according to the invention are shown by the following test:

Pigs are used as test animals. Each animal group consists of 6 pigs and each test with 6 pigs is repeated 3 times. The fodder contains 50 mg/kg of the test compound of the general formula I according to the invention. The animals are fed under identical conditions and all the animal groups receive the same amount of fodder having the same composition. The animals of the control group are fed with the same fodder but without test compound of the formula I according to the invention. The results are summarized in the Table.

**Table**

| Test compound | Average daily weight gain | | Weight of fodder producing 1 kg of weight gain | |
|---|---|---|---|---|
| | g/day | % by weight | kg | % by weight |
| 4-{[4'-(n-Hexyloxy)-3'-(methoxy)-phenyl]-thioxomethyl}-morpholine [Example 1] {according to the invention} | 323.5 | 111.7 | 2.05 | 89.5 |
| Control | 289.5 | 100.0 | 2.29 | 100.0 |

From the above data it can be seen that the weight gain of the animals fed with a fodder containing a compound of the invention is significantly higher than that of the pigs of the control group. At the same time the same weight gain can be achieved with a considerably smaller amount of fodder when a compound of the invention is incorporated into the animal feed. This is a proof of an improved fodder utilization.

A very important advantage of the N-[phenyl-(thioxomethyl)]-morpholine derivatives of the invention is that they are discharged from the animal organism more readily than the known weight-gain increasing compounds and they do not show any mutagenic effect. This fact constitutes a significant advantage when used in animal husbandry.

The toxicity of the compounds of the invention on domestic animals is so low that the compounds are practically non-toxic.

Conveniently the compositions according to the invention are fodder additives or concentrates, premixes or ready-for-use fodders having weight-gain increasing effect.

Advantageously the compositions according to the invention contain the compound(s) of the invention as active ingredient(s) in an amount of 0.0001% to 85% by weight, suitably in admixture with 1 or more inert solid and/or liquid carrier(s) and/or diluent(s) and optionally 1 or more additive(s) generally used in the production of fodder additives and fodders.

The fodder additives and concentrates, premixes and ready-for-use fodders can be prepared by admixing 1 or more compound(s) of the invention with 1 or more edible solid and/or liquid carrier(s) and/or diluent(s) and optionally with 1 or more additive(s) generally used in the production of fodder additives and fodders.

As carrier(s) conveniently any substance(s) of vegetable and/or animal origin applicable in the feeding of animals can be used. For this purpose particularly wheat, barley, bran, maize, soybean, oats, rye or alfalfa can be used in appropriate forms, such as grits, groats or meal, or fish meal, meat meal or bone meal, skimmed milk powder or tapioca meal, or mixtures of these materials can be used as well. One may advantageously use a fiber-free green plant fodder concentrate with high protein content.

As additive(s) e.g. silicic acid, 1 or more antioxidant(s), starch, dicalcium phosphate, calcium carbonate and/or sorbic acid can be used. The starch(es) used may be e.g. wheat, maize and/or potato starch.

As wetting agent(s) e.g. 1 or more nontoxic oil(s), preferably soybean oil, maize oil and/or mineral oil, can be applied. Various alkylene glycols can also be used as wetting agent.

The content of the compositions according to the invention of the compound(s) of the invention as active principle(s) may vary within wide ranges. The fodder additives and concentrates according to the invention advantageously may contain about 5 to 80% by weight, preferably about 10 to 80% by weight, of the active ingredient(s) of the invention. The content of the active ingredient(s) of the invention of the animal fodders ready for use advantageously may be about 0.0001 to 0.04% by weight, preferably about 0.001 to 0.01% by weight. The fodder additives and concentrates of the invention advantageously may contain 1 or more usual vitamin(s), e.g. vitamin(s) A, B₁, B₂, B₃, B₆, B₁₂, E and/or K, and trace element(s), e.g. Mn, Fe, Zn, Cu and/or J, too.

The fodder additives and concentrates according to the invention may be diluted with 1 or more suitable fodder component(s) or incorporated into suitable animal feeds to provide animal feeds ready for use.

The compounds and compositions according to the present invention can be used for increasing the weight gain and improving feed utilization of various domestic animals, such as pigs, lambs, cattle and poultry, particularly pigs. Hence a further subject matter of the invention is the use of the substituted N-[phenyl-(thioxomethyl)]-morpholine derivatives as above defined for the compositions according to the invention for increasing the weight gain and improving feed utilization of domestic animals.

The compounds and compositions according to the invention can be used by a method which comprises feeding the animals with a compound or composition of the invention.

Further details of the present invention are to be found in the following Examples. The melting points appearing in the Examples are not corrected.

### Example 1

### 4-{[-4'-(n-Hexyloxy)-3'-(methoxy)-phenyl]-thioxomethyl}-morpholine

261.3 g (3.0 mole) of morpholine, 48.0 g (1.6 mole) of powdered sulphur and 354.5 g (1.5 mole) of 4-(n-hexyloxy)-3-(methoxy)-benzaldehyde are allowed to react at the boiling point of the mixture for 2 hours. The product is crystallized from ethanol.
Yield: 486.5 g (96.1 %) or yellow lamellated crystals.
M.p.: 105-107 °C.

| Analysis for the formula C₁₈H₂₇NO₃S (337.5): | | | | |
|---|---|---|---|---|
| calculated: | C% = 64.06 | H% = 8.06 | N% = 4.17 | S% = 9.50 |
| found: | C% = 64.28 | H% = 8.14 | N% = 4.20 | S% = 9.44. |

UV: λₘₐₓ = 283 nm (ε = 15599).

### Example 2

### 4-{[4'-(Prop-2''-enyloxy)-3'-(methoxy)-phenyl]-thioxomethyl}-morpholine

174.2 g (2.0 mole) of morpholine, 35.3 g (1.1 mole) of powdered sulphur and 192.1 g (1.0 mole) of 4-(prop-2'-enyloxy)-3-(methoxy)-benzaldehyde are reacted at the boiling point of the reaction mixture for 4 hours. Then ethanol is added to it and the desired product is isolated by crystallization.
Yield: 273.1 g (93.1 %) of yellow powdery crystals.
M.p.: 186-190 °C.

| Analysis for the formula C₁₅H₁₉NO₃S (293.3): | | | | |
|---|---|---|---|---|
| calculated: | C% = 61.40 | H% = 6.52 | N% = 4.77 | S% = 10.92 |
| found: | C% = 61.63 | H% = 6.97 | N% = 4.80 | S% = 10.95: |

UV: λₘₐₓ = 286 nm (ε = 15410).

### Example 3

### 4-{[4'-(Prop-2''-ynyloxy)-3'-(methoxy)-phenyl]-thioxomethyl}-morpholine

63.35 9 (0.25 mole) of 4-{[4'-(hydroxy)-3'-(methoxy)-phenyl]-thioxomethyl}-morpholine are reacted with 20.1 g (0.27 mole) of 3-(chloro)-prop-l-yne in dimethylformamide, in the presence of 27.5 g (0.2 mole) of potassium carbonate at the boiling point of the reaction mixture for 4 hours. The desired compound is then precipitated with water and filtered.
Yield: 63.0 g (86.5 %) of yellow powder
M.p.: 111-113 °C

| Analysis for the formula C₁₅H₁₇NO₃S (291.4): | | | | |
|---|---|---|---|---|
| calculated: | C% = 61.83 | H% = 5.88 | N% = 4.81 | S% = 11.01 |
| found: | C% = 61.94 | H% = 5.99 | N% = 4.82 | S% = 10.94, |

UV: λₘₐₓ = 282 nm (ε = 14101).

The starting compound 4-{[4'-(hydroxy)-3'-(methoxy)-phenyl]-thioxomethyl}-morpholine is prepared as follows:
104.5 g (1.5 mole) of morpholine, 32.0 g (1.0 mole) of powdered sulphur and 152.2 g (1.0 mole) of 4-(hydroxy)-3-(methoxy)-benzaldehyde are reacted at the boiling point of the mixture (115°C) for 4 hours. The product is crystallized from ethanol.
Yield: 226.8 g (89.5 %) of yellow crystals,
M.p.: 167-168 °C.

| Analysis for the formula C₁₂H₁₅NO₃S (253.4): | | | | |
|---|---|---|---|---|
| calculated: | C% = 56.87 | H% = 5.97 | N% = 5.56 | S% = 12.65 |
| found: | C% = 57.02 | H% = 6.05 | N% = 5.49 | S% = 12.66, |

UV: λₘₐₓ = 281 nm (ε = 15442).

### Example 4

### 4-{[4'-(n-Dodecyloxy)-3'-(methoxy)-phenyl]-thioxomethyl}-morpholine

One proceeds according to Example 3 except that instead of 3-(chloro)-prop-1-yne 67.3 g (0.27 mole) of 1-(n-dodecyl)-bromide are used. The desired compound is precipitated with water and filtered.
Yield: 264.9 g (83.8 %) of yellow powder.
M.p.: 104-106 °C.

| Analysis for the formula C₂₄H₃₉NO₃S (421.6): | | | | |
|---|---|---|---|---|
| calculated: | C% = 68.36 | H% = 9.32 | N% = 3.32 | S% = 7.61 |
| found: | C% = 68.40 | H% = 9.45 | N% = 3.34 | S% = 7.61. |

UV: λₘₐₓ = 283 nm (ε = 15517).

### Example 5

### 4-{[4'-(n-Butoxy)-3'(methoxy)-phenyl]-thioxomethyl}--morpholine

One proceeds according to Example 3 except that instead of 3-(chloro)-prop-1-yne 37.0 g (0.27 mole) of 1-(bromo)-n-butane are used.
Yield: 87.9 g (94.7 %) of yellow lamellated crystals.
M.p.: 113-115 °C.

| Analysis for the formula C₁₆H₂₃NO₃S (309.6): | | | | |
|---|---|---|---|---|
| calculated: | C% = 62.10 | H% = 7.49 | N% = 4.52 | S% = 10.34 |
| found: | C% = 62.31 | H% = 7.52 | N% = 4.61 | S% = 10.39. |

UV: λₘₐₓ = 284 nm (ε = 14195).

### Example 6

### 4-{[3'-(Bromo)-4'-(prop-2''-ynyloxy)-5'-(methoxy)-phenyl]-thioxomethyl}-morpholine

One proceeds according to Example 1 except that instead of 354.5 g (1.5 mole) of 4-(n-hexyloxy)-3--(methoxy)-benzaldehyde 403.8 g (1.5 mole) of 3-(bromo)-4-(prop-1'-ynyloxy)-5-(methoxy)-benzaldehyde are used.
Yield: 509.4 g (91.7 %) of yellow crystals.
M.p.: 137-139 °C.

| Analysis for the formula C₁₅H₁₆BrNO₃S (370.4): | | | | | |
|---|---|---|---|---|---|
| calculated: | C% = 48.64 | H% = 4.35 | N% = 3.78 | Br% = 21.58 | S% = 8.66 |
| found: | C% = 48.59 | H% = 4.43 | N% = 3.87 | Br% = 21.63 | S% = 8.80. |

UV: λₘₐₓ = 283 nm (ε = 11976).

### Example 7

### 4-{[3'-(Bromo)-4'-(hydroxy)-5'-(methoxy)-phenyl]-thioxomethyl}-morpholine

One proceeds according to Example 1 except that instead of 354.5 g (1.5 mole) of 4-(n-hexyloxy)-3-(methoxy)-benzaldehyde 346.65 g (1.5 mole) of 3-(bromo)-4-(hydroxy)-5-(methoxy)-benzaldehyde are used.
Yield: 282.8 g (85.1 %) of yellow powder.
Mp.: 197-199 °C.

| Analysis for the formula C₁₂H₁₄BrNO₃S (332.3): | | | | | |
|---|---|---|---|---|---|
| calculated: | C% = 43.37 | H% = 4.24 | Br% = 24.04 | N% = 4.23 | S% = 9.64 |
| found: | C% = 43.22 | H% = 4.39 | Br% = 23.97 | N% = 4.36 | S% = 9.87. |

UV: λₘₐₓ = 284 nm (ε = 14877).

### Example 8

### 4-{[3'-(Bromo)-4'-(n-hexyloxy)-5'-(methoxy)-thioxomethyl}-morpholine

One proceeds according to Example 1 except that instead of 354.5 g (1.5 mole) of 4-(n-hexyloxy)-3-(methoxy)-benzaldehyde 472.8 g (1.5 mole) of 3-(bromo)-4-(n-hexyloxy)-5-(methoxy)-benzaldehyde are used.
Yield: 196.5 g (94.4 %) of yellow crystals.
M.p.: 83-85 °C.

| Analysis for the formula C₁₈H₂₆BrNO₃S (416.4): | | | | | |
|---|---|---|---|---|---|
| calculated: | C% = 51.92 | H% = 6.29 | Br% = 19.19 | N% = 3.36 | S% = 7.69 |
| found: | C% = 52.10 | H% = 6.35 | Br% = 19.19 | N% = 3.40 | S% = 7.74. |

UV: λₘₐₓ = 284 nm (ε = 12796).

### Example 9

A premix for supplementing pig fodder is prepared with the following composition:

| Components | Amounts |
|---|---|
| Vitamin A | 3 000 000 NE |
| Vitamin D₃ | 600 000 NE |
| Vitamin E | 4 000 NE |
| Vitamin K₃ | 400 mg |
| Vitamin B₁ | 600 mg |
| Vitamin B₂ | 800 mg |
| Vitamin B₃ | 2 000 mg |
| Vitamin B₆ | 800 mg |
| Vitamin B₁₂ | 10 mg |
| Niacine | 4 000 mg |
| Choline chloride | 60 000 mg |
| Active principle 4-{[4'-(n-hexyloxy)-3'-(methoxy)-phenyl]-thioxomethyl}-morpholine according to Example 1 | 10 000 mg |
| Butylhydroxytoluene (antioxidant) | 30 000 mg |
| Flavouring substances | 8 000 mg |
| Sodium saccharate | 30 000 mg |

| Trace elements: | |
|---|---|
| Mn | 8 000 mg |
| Fe | 30 000 mg |
| Zn | 20 000 mg |
| Cu | 6 000 mg |
| J | 1 000 mg |
| Twice-ground bran ad | 1 000 g |

This premix of vitamins and trace elements is admixed with the basal fodder having the composition of that of Example 13 in a concentration of 0.5 kg per 100 kg.

### Example 10

A premix for supplementing piglet fodder is prepared with the following composition:

| Components | Amounts |
|---|---|
| Vitamin A | 1 200 000 NE |
| Vitamin D₃ | 300 000 NE |
| Vitamin E | 2 000 NE |
| Vitamin B₂ | 600 mg |
| Vitamin B₃ | 2 000 mg |
| Vitamin B₁₂ | 5 mg |
| Niacine | 3 000 mg |
| Choline chloride | 40 000 mg |
| Active principle 4-{[4'-(n-hexyloxy)-3'-(methoxy)-phenyl]-thioxomethyl}-morpholine according to Example 1 | 10 000 mg |
| Butylhydroxytoluene (antioxidant) | 30 000 mg |

| Trace elements: | |
|---|---|
| Mn | 6 000 mg |
| Fe | 10 000 mg |
| Zn | 15 000 mg |
| Cu | 30 000 mg |
| J | 100 mg |
| Twice-ground bran ad | 1 000 g |

This premix of vitamins and trace elements is admixed with the basal fodder having the composition of that of Example 13 in a concentration of 0.5 kg/100 kg.

### Example 11

0.5 kg of premix as described in Example 9 is admixed with a basal fodder to yield a total weight of 100.0 kg of the following composition:

| Components | Amounts |
|---|---|
| Maize | 37.6 kg |
| Barley | 25.4 kg |
| Wheat | 6.0 kg |
| Oats | 5.0 kg |
| Soybean | 13.0 kg |
| Fish meal | 6.0 kg |
| Bran | 2.4 kg |
| Fat powder | 1.5 kg |
| Premix of minerals | 1.0 kg |
| Lime (fodder quality) | 1.0 kg |
| Sodium chloride (fodder quality) | 0.5 kg |
| Biolysine | 0.1 kg |
| Premix according to Example 9 | 0.5 kg |
| Total weight: | 1̅0̅0̅.̅0̅ k̅g̅ |

The active principle content of the resulting pig fodder is 0.005% by weight.

The composition of the premix of minerals is as follows:

| Components | Amounts, % by weight |
|---|---|
| Dicalcium phosphate | 55.0 |
| Monocalcium phosphate | 40.0 |
| Calcium carbonate | 5.0 |

### Example 12

0.5 kg of a premix as described in Example 10 is admixed with a basal fodder to yield a total weight of 100.0 kg of the following composition:

| Components | Amounts |
|---|---|
| Maize | 25.0 kg |
| Wheat | 34.0 kg |
| Extracted soybean | 18.0 kg |
| Milk powder | 9.9 kg |
| Fish meal | 4.0 kg |
| Yeast (fodder quality) | 2.0 kg |
| Fat powder | 3.4 kg |
| Premix of minerals according to Example 11 | 1.8 kg |
| Lime (fodder quality) | 1.0 kg |
| Sodium chloride (fodder quality) | 0.4 kg |
| Premix according to Example 10 | 0.5 kg |
| Total weight: | 1̅0̅0̅.̅0̅ k̅g̅ |

The acitive principle content of the resulting piglet fodder is 0.005% by weight.

### Example 13

400 kg of a pre-ground soybean meal are filled into a mixer, 3.1 kg of soybean oil are added under stirring, and the mixture is stirred until the solids get coated with oil. Thereafter 9.1 kg of the active principle according to Example 4 are added and the mixture is stirred until a homogeneous blend is obtained. Finally 9.0 kg of soybean oil are added and the mixture is homogenized again.

### Example 14

0.5 kg of the active principle according to Example 3 are added to 40 kg of corn meal under stirring, and simultaneously propylene glycol is sprayed into the mixture. Thereafter 1.4 kg of dicalcium phosphate are added and the mixture is homogenized.

### Example 15

10 kg of alfalfa meal and 15 kg of a fiber-free green plant fodder concentrate with high protein content are stirred for 20 hours, thereafter spraying of 1 kg of maize oil into the mixture is started with an uniform speed so that spraying is continued during the introduction of the following additional components: 2.5 kg of the active principle according to Example 1, 10 kg of maize starch, 0.3 kg of silicon dioxide, 0.6 kg of ascorbic acid and 9 kg of maize starch. The mixture thus obtained is stirred for additional 5 minutes.

### Example 16

One proceeds as described in Example 13 with the difference that 1,4-butylene glycol is applied as wetting agent instead of soybean oil.

### Example 17

A) 3.5 kg of potato starch are admixed with 2.9 kg of the active principle according to Example 8. 0.05 kg of mineral oil are sprayed into the mixture, thereafter 0.2 kg of sorbic acid, 0.4 kg of silicon dioxide and 0.1 kg of calcium propionate are added, and the mixture is stirred for additional 2 minutes.
B) 4.2 kg of fish meal are admixed with 22 kg of rye bran, 0.6 kg of mineral oil are sprayed into the mixture, thereafter 4 kg of a mixture prepared according to point A), 10 kg of maize meal, 4 kg of a mixture prepared according to point A) and 9 kg of maize meal are introduced under stirring. Finally 0.6 kg of mineral oil are sprayed into the mixture.

### Example 18

100 kg of wheat bran, 10 kg of the active principle according to Example 5, 2.5 kg of calcium carbonate, 0.15 kg of α-tocopherol and 0.4 kg of calcium propionate are homogenized with 4 kg of propylene glycol.

### Example 19

10 kg of soybean meal and 0.6 kg of the active principle according to Example 6 are homogenized with 2.5 kg of 1,4-butylene glycol.

### Example 20

50 kg of soybean meal, 6 kg of the active principle according to Example 7, 0.5 kg of silicon dioxide, 1.6 kg of soybean oil and 0.2 kg of calcium propionate are homogenized.

## Claims

1. Substituted N-[phenyl-(thioxomethyl)]-morpholine derivatives of the general formula 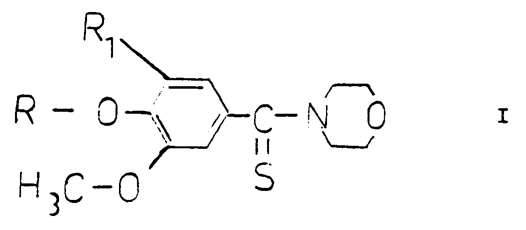 wherein
R represents hydrogen, C₁₋₁₈ alkyl, C₂₋₆ alkenyl or C₂₋₆ alkynyl and
R₁ stands for hydrogen, halogen or methoxy,
with the provisos that
a) at least one of R and R₁ is other than hydrogen,
b) if R represents ethyl
R₁ is other than hydrogen or methoxy,
c) if R stands for methyl
R₁ is other than hydrogen or methoxy and
d) if R stands for hydrogen
R₁ is other than methoxy
and acid addition salts thereof.

2. N-[phenyl-(thioxomethyl)]-morpholine derivatives according to claim 1, characterized in that the C₁₋₁₈ alkyl group which may be represented by R is such having from 4 to 12, particularly from 6 to 12, carbon atoms.

3. N-[Phenyl-(thioxomethyl)]-morpholine derivatives according to claim 1 or 2, characterized in that the C₂₋₆ alkenyl or C₂₋₆ alkynyl group which may be represented by R is such having from 3 to 5, particularly 3 or 4, carbon atoms.

4. N-[Phenyl-thioxomethyl)]-morpholine derivatives according to claims 1 to 3, characterized in that the halogen atom for which R₁ may stand is a bromine or chlorine atom.

5. 4-{[4'-(n-Hexyloxy)-3'-(methoxy)-phenyl]-thioxomethyl}-morpholine.

6. A process for preparing N-[phenyl-(thioxomethyl)]-morpholine derivatives according to claims 1 to 5, characterized by
a) reacting a substituted benzaldehyde of the general formula 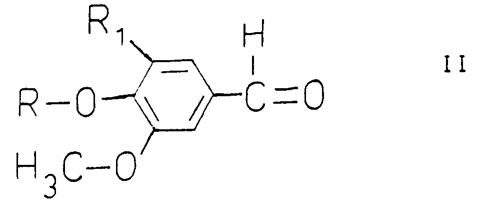 wherein
R and R₁ are as stated in claims 1 to 4, with morpholine and sulphur, or
b) reacting a substituted thiobenzoic acid of the general formula 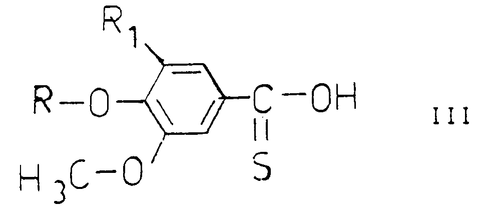 wherein
R and R₁ are as stated in claims 1 to 4, or a reactive derivative thereof with morpholine or
c) reacting a morpholide of the general formula 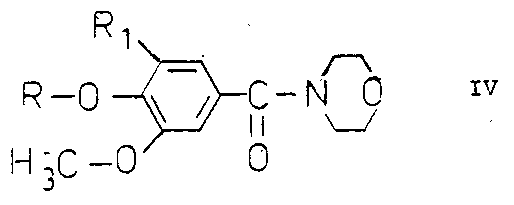 wherein
R and R₁ are as stated in claims 1 to 4,
with a thionating agent and, if desired, reacting a compound of the general formula I, wherein R stands for hydrogen, obtained according to any of process variants a) to c) with a halogenide of the general formula
R₂-Hlg V
wherein
Hlg represents halogen and
R₂ stands for C₁₋₁₈ alkyl, C₂₋₆ alkenyl or C₂₋₆ alkynyl,
and/or, if desired, in a manner known per se, converting the obtained compound of the general formula I into an acid addition salt or if desired, converting the acid addition salt of the compound of formula I into the free base of formula I or into another acid addition salt.

7. Compositions for use in animal husbandry, characterized in that they contain as [an] active ingredient(s) 1 or more substituted N-[phenyl-(thioxomethyl)]-morpholine derivative(s) of the general formula 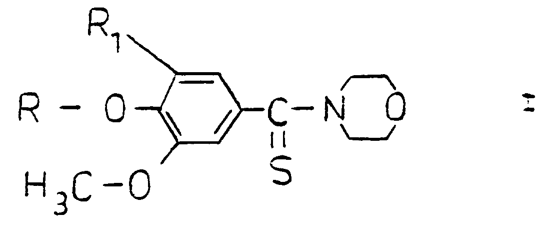 wherein
R represents hydrogen C₁₋₁₈ alkyl, C₂₋₆ alkenyl or C₂₋₆ alkynyl and
R₁ stands for hydrogen, halogen or methoxy,
with the provisos that
a) at least one of R and R₁ is other than hydrogen,
b) if R represents ethyl
R₁ is other than hydrogen and
c) if R stands for methyl
R₁ is other than hydrogen or methoxy,
and acid addition salts thereof suitably in admixture with 1 or more inert solid and/or liquid carrier(s) and/or diluent(s) and optionally also 1 or more additive(s) generally used in the production of fodder additives and fodders.

8. Compositions according to claim 7, characterized in that they are fodder additives or concentrates, premixes or ready-for-use fodders having weight-gain increasing effect.

9. Compositions according to claim 7 or 8, characterized in that they contain as carrier(s) such one(s) of vegetable and/or animal origin applicable in the feeding of animals and/or serving as fodder, particularly wheat, barley, bran, maize, soybean, oats, rye or alfalfa in the form of grits, groats or meal or fish meal, meat meal, bone meal, skimmed milk powder or tapioca meal or mixtures of these materials.

10. Use of the substituted N-[phenyl-(thioxomethyl)]-morpholine derivatives as defined in claim 7 for increasing the weight gain and improving feed utilization of domestic animals.

## Patentansprüche

1. Substituierte N-[Phenyl-(thioxomethyl)]-morpholinderivate der allgemeinen Formel 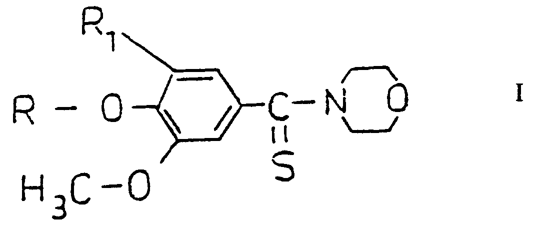 worin
R für ein Wasserstoffatom, C₁₋₁₈-Alkyl, C₂₋₆-Alkenyl oder C₂₋₆-Alkynyl steht und
R₁ für Wasserstoff, Halogen oder Methoxy steht, mit den Maßgaben, daß
a) mindestens eines von R und R₁ etwas anderes als Wasserstoff bedeutet,
b) wenn R für Ethyl steht, R₁ nicht Wasserstoff oder Methoxy ist,
c) wenn R für Methyl steht, R₁ nicht Wasserstoff oder Methoxy ist und
d) wenn R für Wasserstoff steht, R₁ nicht Methoxy ist,
und Säureadditionssalze davon.

2. N-[Phenyl-(thioxomethyl)]-morpholinderivate gemäß Anspruch 1, **dadurch gekennzeichnet,** daß die C₁₋₁₈-Alkylgruppe, welche durch R angegeben werden kann, 4 bis 12, insbesondere 6 bis 12, Kohlenstoffatome besitzt.

3. N-[Phenyl-(thioxomethyl)]-morpholinderivate gemäß Anspruch 1 oder 2, **dadurch gekennzeichnet**, daß die C₂₋₆-Alkenylgruppe oder C₂₋₆-Alkynylgruppe, welche durch R angegeben werden kann, 3 bis 5, insbesondere 3 oder 4, Kohlenstoffatome besitzt.

4. N-[Phenyl-(thioxomethyl)]-morpholinderivate gemäß den Ansprüchen 1 bis 3, **dadurch gekennzeichnet**, daß das Halogenatom, für welches R₁ stehen kann, ein Brom- oder Chloratom ist.

5. 4-{[4'-(n-Hexyloxy)-3'-(methoxy)-phenyl]-thioxomethyl}-morpholin.

6. Verfahren zur Herstellung von N-[Phenyl-(thioxomethyl)]-morpholinderivaten gemäß den Ansprüchen 1 bis 5, **gekennzeichnet durch** das
a) Umsetzen eines substituierten Benzaldehydes der allgemeinen Formel 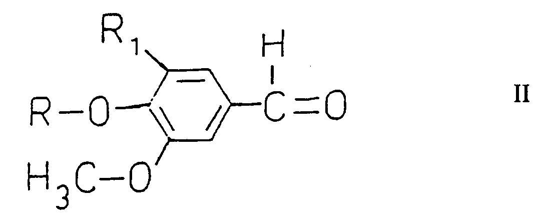 worin R und R₁ in den Ansprüchen 1 bis 4 genannt sind,
mit Morpholin und Schwefel,
b) Umsetzen einer substituierten Thiobenzoesäure der allgemeinen Formel 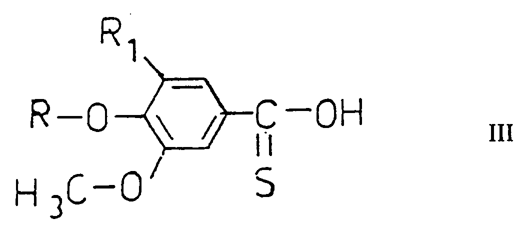 worin R und R₁ in den Ansprüchen 1 bis 4 genannt sind,
oder eines reaktiven Derivates davon mit Morpholin oder
c) Umsetzen eines Morpholids der allgemeinen Formel 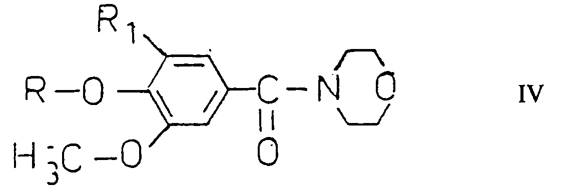 worin R und R₁ in den Ansprüchen 1 bis 4 genannt sind, mit einem thiolierenden Mittel
und, falls gewünscht,
Umsetzen einer Verbindung der allgemeinen Formel I, worin R für Wasserstoff steht, die gemäß einer beliebigen Verfahrensvariante von a) bis c) erhalten worden ist, mit einem Halogenid der allgemeinen Formel
R₂ - Hlg V
worin
Hlg ein Halogen bedeutet und
R ₂ für C₁₋₁₈-Alkyl, C₂₋₆-Alkenyl oder C₂₋₆-Alkynyl steht,
und/oder, falls gewünscht, das Umwandeln der erhaltenen Verbindung der allgemeinen Formel I in ein Säureadditionssalz in einer per se bekannten Weise oder, falls gewünscht, das Umwandeln des Säureadditionssalzes der Verbindung der Formel I in die freie Base der Formel I oder in ein anderes Säureadditionssalz.

7. Zusammensetzungen zur Anwendung in der Tierhaltung, **dadurch gekennzeichnet**, daß sie als (einen) aktive(n) Bestandteil(e) 1 oder mehrere substituierte(s) N-[Phenyl-(thioxomethyl)]-morpholinderivat(e) der allgemeinen Formel 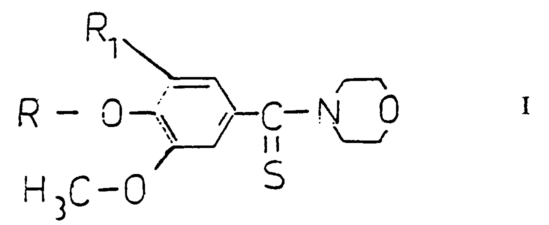 worin
R für ein Wasserstoffatom, C₁₋₁₈-Alkyl, C₂₋₆-Alkenyl oder C₂₋₆-Alkynyl steht und
R₁ für Wasserstoff, Halogen oder Methoxy steht,
mit den Maßgaben, daß
a) mindestens eines von R und R₁ etwas anderes als Wasserstoff bedeutet,
b) wenn R für Ethyl steht, R₁ nicht Wasserstoff ist,
c) wenn R für Methyl steht, R₁ nicht Wasserstoff oder Methoxy ist,
und Säureadditionssalze davon, geeigneterweise in Vermischung mit 1 oder mehreren inerten Feststoffen und/oder flüssigen Trägern und/oder Verdünnungsmitteln, und auch 1 oder mehrere Additive, welche allgemein bei der Herstellung von Futteradditiven und Futterstoffen Verwendung finden, enthalten.

8. Zusammensetzungen gemäß Anspruch 7, **dadurch gekennzeichnet**, daß sie Futteradditive oder -konzentrate, Vormischungen oder gebrauchstfertige Futtermittel mit einer gewichtssteigernden Wirkung sind.

9. Zusammensetzungen gemäß Anspruch 7 oder 8, **dadurch gekennzeichnet**, daß sie als Träger solche(n) enthalten, die (der) von pflanzlichem und/oder tierischem Ursprung sind (ist), die in der Fütterung von Tieren anwendbar sind und/oder als Futter dienen, insbesondere Weizen, Gerste, Kleie, Mais, Sojabohnen, Hafer, Roggen oder Alfalfa bzw. Luzerne in Form von (Spitz)körnern, Grieß oder Pulvern oder Fleischpulver, Knochenpulver, Magermilchpulver oder Tapiokapulver oder Mischungen von diesen Materialien.

10. Verwendung von substituierten N-[Phenyl-(thioxomethyl)]-morpholinderivaten gemäß Anspruch 7 zur Steigerung der Gewichtszunahme und zur Verbesserung der Futterverwertung von Haustieren.

## Revendications

1. Dérivés de N-[phényl-(thioxométhyl)]-morpholine substitués présentant la formule générale 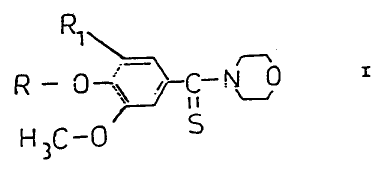 dans laquelle:
R est un atome d'hydrogène, un radical alkyle en C₁₋₁₈, alkényle en C₂₋₆ ou alkynyle en C₂₋₆ et
R₁ est un atome d'hydrogène, un atome d'halogène ou un radical méthoxy,
étant entendu que
a) un au moins des substituants R et R₁ n'est pas un atome d'hydrogène, et
b) si R représente un radical éthyle, R₁ n'est ni un atome d'hydrogène, ni un groupe méthoxy,
c) si R est un radical méthyle, R₁ n'est ni un atome d'hydrogène ni un radical méthoxy,
d) si R est un atome d'hydrogène, R₁ est autre qu'un radical méthoxy,
ainsi que les sels d'addition d'acide en dérivant.

2. Dérivés de N-[phényl-(thioxométhyl)]-morpholine selon la revendication 1, caractérisés en ce que le groupe alkyle en C₁₋₁₈ qui peut être représenté par R est tel qu'il comporte de 4 à 12 et notamment de 6 à 12 atomes de carbone.

3. Dérivés de N-(phényl-(thioxométhyl)]-morpholine selon la revendication 1 ou 2, caractérisés en ce que les radicaux alkényle en C2-6 ou alkynyle en C₂₋₆ qui peuvent être représentés par R sont tels qu'ils comportent de 3 à 5 et notamment 3 à 4 atomes de carbone.

4. Dérivés de N-[phényl-(thioxométhyl)]-morpholine selon les revendications 1 à 3, caractérisés en ce que l'atome d'halogène qui peut être représenté par R1 est un atome de chlore ou un atome de brome.

5. 4-{[4'-(n-Hexyloxy)-3'-(méthoxy)-phényl]-thioxométhyl}-morpholine.

6. Un procédé de préparation de dérivés de N-[phényl-(thioxométhyl)]-morpholine selon les revendications 1 à 5, caractérisé en ce qu'il comprend
a) la réaction d'un benzaldéhyde substitué de formule générale 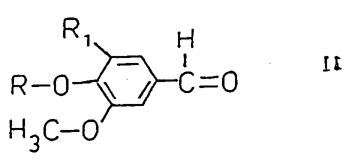 dans laquelle R et R1 sont tels qu'indiqués dans les revendications 1 à 4,
avec morpholine et soufre,
b) la réaction d'un acide thiobenzoïque substitué de formule générale 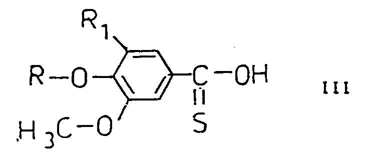 dans laquelle R et R1 sont tels qu'indiqués dans les revendications 1 à 4, ou un de ses dérivés réactifs avec la morpholine ou
c) réaction d'un morpholide de formule générale 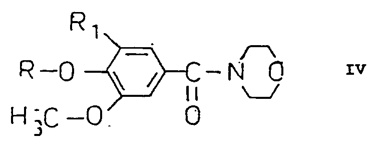 dans laquelle R et R1 sont tels qu'indiqués dans les revendications 1 à 4, avec un agent de thionation et le cas échéant réaction d'un composé de formule générale I, dans laquelle R est un atome d'hydrogène obtenu selon l'une des variantes de procédé a) à c) du procédé avec un halogénure de formule générale
R₂-Hlg V
dans laquelle
Hlg représente un halogène et
R₂ est un radical alkyle en C₁₋₁₈, alkényle en C₂₋₆ ou alkynyle en C₂₋₆,
et/ou, le cas échéant, et d'une façon connue en soi, la conversion du composé obtenu de formule générale I en un sel d'addition d'acide ou encore, le cas échéant, conversion du sel d'addition d'acide du composé de formule I en la base libre de formule I ou en un autre sel d'addition d'acide.

7. Compositions destinées à être utilisées dans l'élevage, caractérisées en ce qu'elles contiennent, en tant qu'ingrédient(s) actif(s) un ou plusieurs dérivé(s) N-[phényl-(thioxométhyl)]-morpholine substitués de formule générale 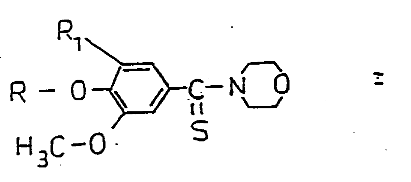 dans laquelle
R est un atome d'hydrogène ou un radical alkyle en C₁₋₁₈, alkényle en C₂₋₆ ou alkynyle en C₂₋₆ et
R1 est un atome d'hydrogène, un atome d'halogène ou un radical méthoxy, étant entendu que
a) au moins des substituants R et R1 n'est pas un atome d'hydrogène, et
b) si R représente un radical éthyle, R1 n'est pas un atome d'hydrogène, et
c) si R est un radical méthyle, R1 n'est ni un atome d'hydrogène ni un radical méthoxy,
ainsi que les sels d'addition d'acide en dérivant, convenablement associés avec un ou plusieurs diluant(s) et/ou support(s) solide(s) et/ou liquide(s), éventuellement également un ou plusieurs additif(s) du type généralement utilisés dans la fabrication d'additifs de fourrage et de produits d'affourragement.

8. Compositions selon la revendication 7, caractérisée en ce qu'elles consistent en additifs de fourrage ou produits d'affourragement concentrés, prémélangés ou prêts à l'emploi, présentant un effet d'augmentation de poids.

9. Compositions selon la revendication 7 ou 8, caractérisées en ce qu'elles contiennent en tant que support(s) un ou plusieurs composés d'origine végétale et/ou animale utilisable(s) pour l'alimentation des animaux et/ou pouvant être utilisé(s) en tant que produit d'affourragement, notamment froment, orge, son, maïs, soja, avoine, seigle ou alfalfa, sous forme de granulés, gruau, farine ou farine de poisson, farine de viande, farine d'os, poudre de lait écrémé ou farine de tapioca ou des mélanges de ces matières.

10. Utilisation du dérivé de N-[phényl-(thioxométhyl)]-morpholine substitué tel que défini dans la revendication 7, pour augmenter le poids et améliorer l'utilisation des aliments pour les animaux domestiques.
